# FASCICULE DE BREVET EUROPEEN

(11) **EP 0 579 991 B1**
(45) Date de publication et mention de la délivrance du brevet: **16.09.1998**
(21) Numéro de dépôt: 93110494.7
(22) Date de dépôt: 01.07.1993
(51) Int. Cl.: C07C 49/447, C07C 45/67, C07C 69/732, C07F 9/117, C07C 69/757

(54) **Procédé pour la préparation d'une cétone bicyclique décalinique**
Verfahren zur Herstellung eines bicyclischen Decalinketons
Method for the preparation of a bicyclic decalin ketone

(30) Priorité: 24.07.1992 CH 2341/92
(43) Date de publication de la demande: 26.01.1994
(73) Titulaire: FIRMENICH SA, CH-1211 Genève 8 (CH)
(72) Inventeur: Snowden, Roger Leslie, Dr., F-74580 Viry (FR); Mahaim, Cyril, Dr., CH-1112 Echichens (CH); Simmons, Dana P., Dr., Jamestown, North Carolina 27282 (US)
(74) Mandataire: Salvaterra-Garcia, Maria de Lurdes

(56) Documents cités:
- TETRAHEDRON, SUPPLEMENT NO. 1, vol. 37, 1981 pages 303-317, F.W. SUM ET AL
- J. CHEM. SOC. CHEM. COMM., no.6, 1992 pages 503-505, S.R. HARRING ET AL
- HELV. CHIM. ACTA, vol. 73, no.7, 1990 pages 1935-1947, S. ESCHER ET AL
- HELV. CHIM. ACTA, vol. 72, no.5, 1989 pages 996-1000, G. BÜCHI ET AL
- BULL. SOC. CHIM. BELG., vol. 95, no.9-10, 1986 pages 771-781, K. MORI ET AL
- TETRAHEDRON LETTERS, vol. 34, no.30, 1993 pages 4789-4792, S.M. LINDER ET AL

## Description

La présente invention a trait au domaine de la synthèse organique, plus particulièrement elle concerne un procédé pour la préparation d'une cétone bicyclique décalinique, la perhydro-5,5,8a-triméthyl-2-naphtalénone. Cette cétone représente un composé intermédiaire utile dans la synthèse de l'acétate de perhydro-5,5,8a-triméthyl-2-naphtalényle, [Polywood®, origine : Firmenich SA], spécialité de parfumerie fort appréciée pour ses qualités odorantes et sa note boisée tenace et élégante. La cétone elle même possède une odeur forte, boisée et ambrée. Compte tenu de sa structure, elle peut se présenter sous deux formes isomériques de formule 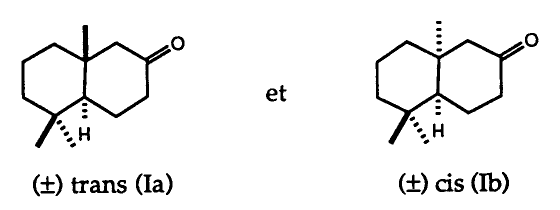 leur réduction suivie d'acétylation conduisant à l'acétate désiré.

A l'expérience il s'est avéré que, parmi les isomères possibles, l'acétate de perhydro-5,5,8aα-triméthyl-2α-trans-naphtalényle est celui des composés qui possède les caractères olfactifs les plus intéressants, l'isomère 2β développant quant à lui une note moins riche, quoique toujours boisée et ambrée.

Le brevet FR 1 593 814 décrit une méthode de préparation des cétones (Ia,b), méthode qui fait appel à l'oxydation d'un carbinol décalinique, lequel est obtenu comme indiqué ci-après.

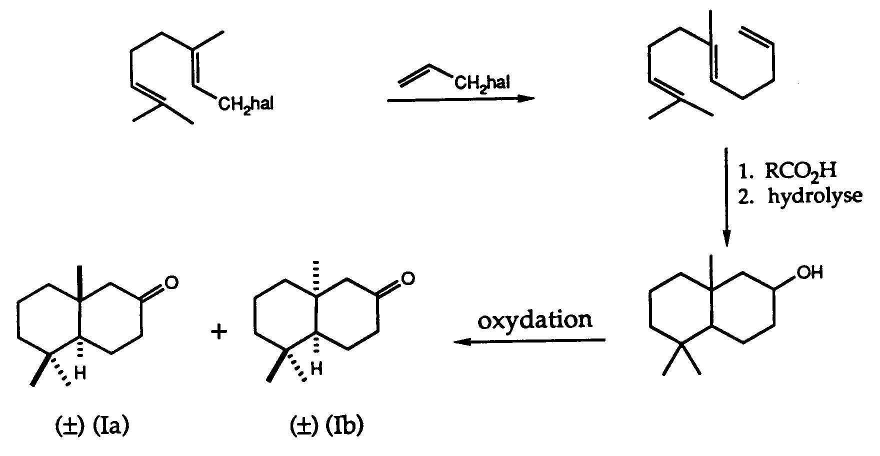

Compte tenu de ce qui précède, nos efforts se sont portés sur la préparation de la perhydro-5,5,8a-triméthyl-2-naphtalénone sous sa forme isomérique trans.

La présente invention apporte une solution à ce problème.

L'un des objets de la présente invention est en effet un procédé pour la préparation de perhydro-5,5,8a-triméthyl-2-naphtalénone essentiellement sous la forme isomérique (Ia), lequel procédé est caractérisé en ce qu'on traite avec un agent de cyclisation acide
a. un ester carboxylique de formule 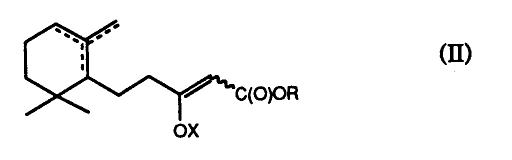 possédant une double liaison dans l'une des positions indiquées par les pointillés et dans laquelle le symbole R définit un groupe alkyle C₁-C₆, X sert à désigner un radical monovalent de formule P(O)(OR¹)₂ ou C(O)R², R¹ étant un groupe alkyle C₁-C₆ et R² représentant soit un groupe alkyle linéaire ou ramifié C₁-C₆, soit un groupe phényle substitué ou non, et dans laquelle la ligne ondulée sert à définir une liaison C-C de configuration cis ou trans,
   ou
b. un ester carboxylique de formule 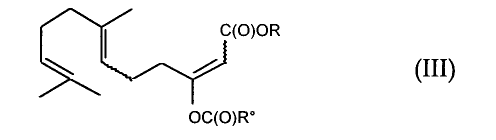 dans laquelle les lignes ondulées et le symbole R sont définis comme indiqués plus haut, et R⁰ représente un radical alkyle C₃-C₆, de préférence ramifié,
et qu'on décarboxyle ensuite le produit de cyclisation ainsi obtenu au moyen d'un traitement avec une base.

La publication J. Chem. Soc. Chem. Commun. 1992, 503 décrit la cyclisation d'un composé de formule

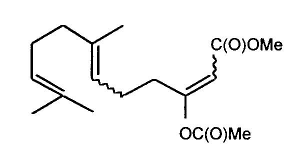

Le produit obtenu après cyclisation est susceptible de donner le produit selon les formules (Ia) et (Ib), après une réaction de décarboxylation.

La cylisation est effectuée à l'aide du BF₃.MeNO₂. La réaction donne un rendement bas et est effectuée à -20°C. Cette réaction, cependant, avance seulement avec un acide de Lewis.

La décarboxylation du produit de cyclisation est décrite dans Helv. Chim. Acta 73, 1935 (1990). Dans cette publication, on utilise d'autres produits de départ pour la cyclisation que ceux de la présente invention.

A titre d'agent de cyclisation acide on peut employer un acide protique, minéral ou organique, ou un acide du type dit de Lewis. A cet effet on peut utiliser l'acide sulfurique, phosphorique, chlorosulfonique, p-toluènesulfonique ou formique, par exemple, tandis que parmi les acides de Lewis il convient de mentionner le BF₃. La cyclisation peut également être effectuée à l'aide de résines acides échangeuses d'ions,

En pratique, il est apparu que la réaction de cyclisation s'effectuait avec les rendements les meilleurs lorsque la proportion de l'acide correspondait à environ 2 équivalents par rapport à l'ester de départ. Des proportions allant au-delà de cette valeur peuvent cependant être utilisées sans que cela ait une influence marquée sur les rendements observés.

En ce qui concerne la température, la cyclisation est conduite à 0-25° C, de préférence à 5-10° C.

La réaction suivante de décarboxylation peut s'effectuer selon les techniques usuelles grâce à l'emploi des réactifs connus, par exemple d'une base tel un hydroxyde de métal alcalin comme l'hydroxyde de potassium, de préférence en solution alcoolique ou aqueuse-alcoolique, ou l'hydroxyde de sodium.

A titre de produit de départ de formule (II) et (III) on emploie un ester carboxylique d'alkyle choisi, par exemple, parmi les esters alkyliques de l'acide
5-(2,6,6-triméthyl-1-cyclohexén-1-yl)-3-(diéthoxyphosphoroxy)-pent-2-énoïque,
5-(2,6,6-triméthyl-1-cyclohexén-1-yl)-3-propionoxypent-2-énoïque,
5-(2,6,6-triméthyl-1-cyclohexén-1-yl)-3-(2-méthylpropionoxy)-pent-2-énoïque,
5-(2,6,6-triméthyl-1-cyclohexén-1-yl)-3-(2,2-diméthylpropionoxy)-pent-2-énoïque,
5-(2,6,6-triméthyl-1-cyclohexén-1-yl)-3-benzoyloxypent-2-énoïque,
ainsi que leurs isomères du type 5-(2,6,6-triméthyl-2-cyclohexén-1-ylique), et les esters alkyliques de l'acide
7,11-diméthyl-3-(2,2-diméthyl-propionoxy)-dodéca-2,6,10-triénoïque.

Il s'agit de préférence d'esters méthyliques, éthyliques, propyliques, les premiers étant préférés.

Comme indiqué plus haut, ces composés peuvent se présenter sous une forme de configuration cis ou trans, compte tenu de la position relative du groupe carboxylique par rapport au groupe OX (composés II) ou OC(O)R⁰ (composés III).

Nous avons remarqué que les rendements les meilleurs étaient obtenus par la cyclisation des esters de configuration trans et que par conséquent le procédé s'effectue de préférence avec ce type de composés ou avec des mélanges dans lesquels le contenu en isomère trans est prépondérant. Il en va de même en ce qui concerne la sélectivité, le contenu en l'isomère trans-du perhydro-5,5,8a-triméthyl-2-naphtalénone (composé Ia) étant supérieur lorsque la cyclisation est conduite sur les esters d'isomérie trans.

Les esters de formule (II), à l'exception du composé (Z)-5-(2,6,6-triméthyl-1-cyclohexén-1-yl)-3-(diéthoxyphosphoroxy)-pent-2-énoate de méthyle, sont des composés nouveaux et à ce titre ils constituent également un des objets de la présente invention.

Ce composé est décrit dans le document Tetrahedron 37, 303 (1981), malgré qu'il ne soit ni isolé ni caractérisé.

Ils peuvent être obtenus à partir de carboalkoxy-dihydro-ionones par une méthode simple par analogie avec des techniques connues, tel qu'il apparaît dans le schéma réactionnel que voici

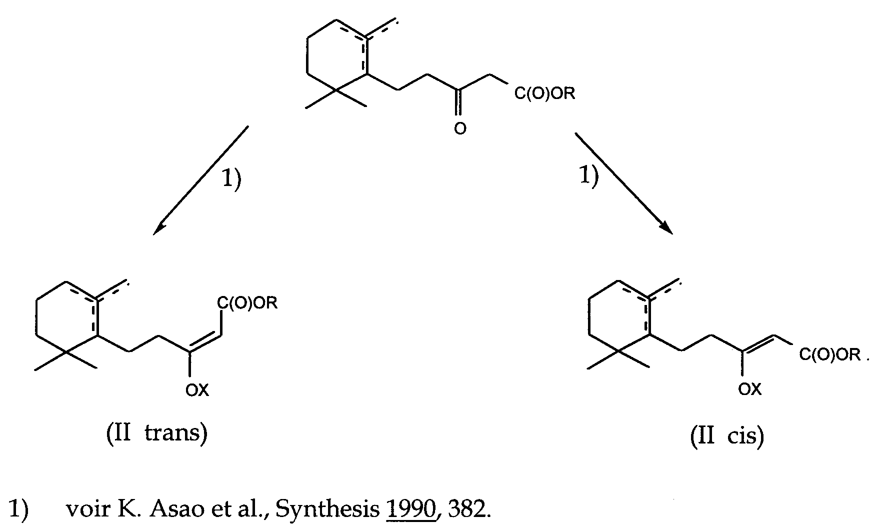

Par analogie, les esters (III) peuvent être obtenus à partir de carboalkoxygéranylacétone

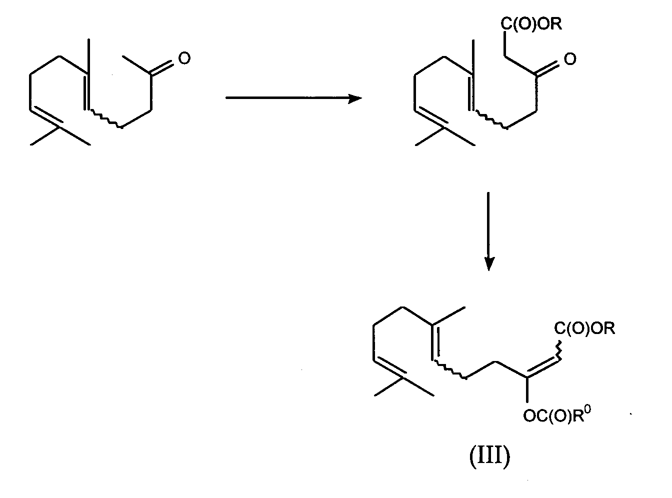

A l'exception du composé (III) pour lequel R⁰=R=CH₃ [voir Chem. Commun. 1992, 503, cité auparavant], les composés (III) sont des composés nouveaux et constituent un autre objet de la présente invention.

Les exemples qui suivent sont destinés à donner une idée plus précise sur la portée et la mise en oeuvre de l'invention. Les températures figurant dans les exemples sont indiquées en degrés centigrades.

### Exemple 1

Les esters carboxyliques de formule (II) et (III) ont été cyclisés par la méthode générale décrite ci-après.
1 G d'ester de départ dans 1 mi de toluène a été ajouté goutte à goutte à 2° à un mélange de 2 mole-équivalents d'acide sulfurique aqueux à 98% dans 9 ml de toluène.
Après 2 h, le mélange de réaction a été versé dans une solution aqueuse saturée de bicarbonate de sodium et extrait au toluène.
L'étape suivante de décarboxylation a été effectuée sur le céto-ester ainsi obtenu par un traitement avec un hydroxyde alcalin, par exemple avec du NaOH.

Le tableau ci-après résume les résultats obtenus par la conversion des esters carboxyliques indiqués

| | cétone décalinique | | rendement [%] |
|---|---|---|---|
| | Ia | Ib | |
| (a) | 98 | 2 | 81 |
| (b) | 95 | 5 | 72 |
| (c) | 95 | 5 | 49 |
| (d) | 95 | 5 | 61 |
| (e) | 97 | 3 | 79 |
| (f) | 96 | 4 | 70 |
| (a)= (E)-5-(2,6,6-triméthyl-1-cyclohexén-1-yl)-3-(diéthoxyphosphoroxy)-pent-2-énoate de méthyle (b)= (Z)-5-(2,6,6-triméthyl-1-cyclohexén-1-yl)-3-(diéthoxyphosphoroxy)-pent-2-énoate de méthyle (c)= 5-(2,6,6-triméthyl-1-cyclohexén-1-yl)-3-propionoxypent-2-énoate de méthyle (d)= 5-(2,6,6-triméthyl-1-cyclohexén-1-yl)-3-(2-méthylpropionoxy)-pent-2-énoate de méthyle (e)= 5-(2,6,6-triméthyl-1-cyclohexén-1-yl)-3-(2,2-diméthylpropionoxy)-pent-2-énoate de méthyle (f)= 5-(2,6,6-triméthyl-1-cyclohexén-1-yl)-3-benzoyloxypent-2-énoate de méthyle | | | |

La préparation desdits esters de départ a été effectuée ainsi :

### Préparation du composé (a)

2,3 G (12,9 mM) de diéthylchlorophosphate ont été ajoutés sous azote à 2° à une solution maintenue sous agitation de 2,5 g (9,4 mM) de 5-(2,6,6-triméthyl-1-cyclohexén-1-yl)-3-oxopentanoate de méthyle et 1,3 g (12,8 mM) de triéthylamine dans 25 ml de N-méthyl-pyrrolidone contenant 0,04 g (0,3 mM) de 4-(N,N-diméthylamino)-pyridine. Après 1 h à température ambiante, le mélange de réaction a été versé dans une solution aqueuse saturée de NH₄Cl et extrait à l'éther. L'évaporation des extraits éthérés combinés séchés a fourni un résidu qui par séparation chromatographique sur colonne (SiO₂, cyclohexane/AcOEt 1,5:1) et distillation sous vide à donné une huile jaunâtre (2,4 g, 65%).
- Eb. (four à boules) :: 200-220°/6,6 Pa.
- R_{f}(cyclohexane/AcOEt 1,5:1) :: 0,36.
- IR(CHCl₃) :: 2933, 1717, 1646, 1437, 1371, 1274, 1162, 1126, 1036.
- RMN(¹H) :: 1,04(s, 6H); 1,39(t, J=7, 6H); 1,43(2H); 1,58(2H); 1,69(s, 3H); 1,93(t, J=6, 2H); 2,27(2H); 2,85(2H); 3,71(s, 3H); 4,22(dq, J=7, 7, 4H); 5,86(large s, 1H) δ ppm.
- RMN(¹³C) :: 166,7(8)(s); 135,8(s); 128,7(s); 104,6(d); 64,8(9)(t); 51,3(q); 39,9(t); 35,1(s); 32,9(t); 32,6(t); 28,5(q); 25,8(t); 19,7(q); 19,5(t); 16,1(q) δ ppm.
- SM :: 388(5, M⁺), 252(41), 220(69), 192(29), 155(100), 127(37), 99(65).

### Préparation du composé (b)

Une solution de 2,5 g (9,4 mM) de 5-(2,6,6-triméthyl-1-cyclohexén-1-yl)-3-oxopentanoate de méthyle dans 10 ml de tétrahydrofurane (THF) a été ajoutée goutte à goutte à température ambiante à un mélange sous agitation de NaH (80% de dispersion dans l'huile; 0,3 g, 0,01 M) dans 25 ml de THF.
Après 1 h à température ambiante, 2,3 g (12,9 mM) de diéthylchlorophosphate ont été introduits goutte à goutte dans le mélange. Après 50 min, le mélange de réaction refroidi a été versé avec précaution dans une solution aqueuse saturée froide de NH₄Cl et extrait à l'éther.
Les traitements usuels ont fourni un résidu qui par chromatographie sur colonne (SiO₂; cyclohexane/AcOEt 2:1) suivi de distillation a donné 3,1 g (rend. 84%) d'une huile incolore.
- Eb. (four à boules) :: 200-220°/6,6 Pa.
- R_{f}(cyclohexane/AcOEt 1,5:1) :: 0,29.
- IR(CHCl₃) :: 2934, 1725, 1664, 1436, 1274, 1201, 1149, 1032.
- RMN(¹H) :: 1,00(s, 6H); 1,37(t, J=7, 6H); 1,41(2H); 1,57(2H); 1,61(s, 3H); 1,91(large t, J=7, 2H); 2,26(2H); 2,48(2H); 3,71(s, 3H); 4,27(dq, J=7,7, 4H); 5,41(s, 1H) δ ppm.
- RMN(¹³C) :: 164,4(s); 162,5(s); 162,4(s); 135,5(s); 128,8(s); 104,5(6)(t); 64,7(8)(t); 51,1(q); 39,9(t); 35,7(t); 35,0(s); 32,8(t); 28,5(2q); 28,1(t); 19,8(q); 19,5(t); 16,1(2q) δ ppm.
- SM :: 388(1, M⁺), 252(100), 220(68), 192(28), 155(53), 99(48).

### Préparation du composé (c)

1,0 G (10,8 mM) de chlorure de propionyle a été ajouté à température ambiante goutte à goutte en 15 min à une solution sous agitation de 2,5 g (9,4 mM) de 5-(2,6,6-triméthyl-1-cyclohexén-1-yl)-3-oxopentanoate de méthyle et 1,1 g (10,8 mM) de triéthylamine dans 30 ml de toluène. Après 2 h à température ambiante, le mélange a été versé dans une solution aqueuse saturée de NH₄Cl et extrait au toluène. Les traitements usuels, suivis de distillation sous vide, ont fourni le composé désiré sous forme d'un mélange isomérique trans/cis 1:1 (huile visqueuse incolore; 1,6 g; rend. 55%).
- Eb. :: 125-140°/33 Pa.
- IR(CHCl₃) :: 2940, 1760, 1718, 1436, 1360, 1230, 1138.

L'isomère trans a montré les caractéristiques suivantes :
- R_{f} (cyclohexane/AcOEt 9:1) :: 0,45.
- RMN(¹H) :: 1,02(s, 6H); 1,22(t, J=7, 3H); 1,42(2H); 1,56(2H); 1,65(s, 3H); 1,91(2H); 2,19(2H); 2,49(q, J=7, 2H); 2,86(2H); 3,72(s, 3H); 5,67(s, 1H) δ ppm.
- RMN(¹³C) :: 171,4(s); 167,1(s); 166,2(s); 136,0(s); 128,6(s); 109,2(d); 51,3(q); 39,9(t); 35,1(s); 32,8(t); 32,0(t); 28,5(2q); 27,7(t); 25,7(t); 19,7(q); 19,5(t); 8,9(q) δ ppm.
- SM :: 308(0, M⁺), 219(8), 137(90), 95(48), 81(35), 57(100).

L'isomère cis était caractérisé par les paramètres analytiques suivants :
- R_{f} (cyclohexane/AcOEt 9:1) :: 0,36.
- RMN(¹H) :: 0,98(s, 6H); 1,24(t, J=7, 3H); 1,42(2H); 1,56(2H); 1,59(s, 3H); 1,91(2H); 2,20(2H); 2,32(2H); 2,57(q, J=7, 2H); 3,68(s, 3H); 5,63(s, 1H) δ ppm.
- RMN(¹³C) :: 171,7(s); 164,5(s); 164,0(s); 135,6(s); 128,5(s); 106,5(d); 51,2(q); 39,8(t); 36,1(s); 35,0(s); 32,9(t); 28,5(2q); 27,9(t); 25,2(t); 19,6(q); 19,5(t); 8,0(q) δ ppm.
- SM :: 308(0, M⁺), 219(12), 137(49), 95(41), 81(31), 57(100).

Le composé (d) a été préparé selon le même procédé que celui décrit ci-dessus en utilisant comme réactif le chlorure de 2-méthylpropionyle. Le composé (d) a été obtenu ainsi sous forme d'un mélange isomérique trans/cis (3,3:1) : 2,0 g; rend. 66%.
- Eb. :: 130-136°/40 Pa.
- IR(CHCl₃) :: 2936, 1752, 1718, 1662, 1437, 1361, 1232, 1099.

Isomère trans :
- R_{f} (cyclohexane/AcOEt 9:1) :: 0,48.
- RMN(¹H) :: 1,02(s, 6H); 1,26(d, J=7, 6H); 1,42(2H); 1,56(2H); 1,65(s, 3H); 1,91(2H); 2,19(2H); 2,69(m, 1H); 2,86(2H); 3,72(s, 3H); 5,65(s, 1H) δ ppm.
- RMN(¹³C) :: 174,3(s); 167,2(s); 166,2(s); 135,9(s); 128,6(s); 109,1(d); 51,3(q); 40,0(t); 35,1(s); 34,4(d); 32,9(t); 31,9(t); 28,5(2q); 25,7(t); 19,7(q); 19,5(t); 16,9(2q) δ ppm.
- SM :: 322(4, M⁺), 234(9), 219(7), 137(81), 71(100).

Isomère cis :
- R_{f}(cyclohexane/AcOEt 9:1) :: 0,39.
- RMN(¹H) :: 0,98(s, 6H); 1,29(d, J=7, 6H); 1,42(2H); 1,56(2H); 1,58(s, 3H); 1,91(2H); 2,19(2H); 2,29(2H); 2,78(m, 1H); 3,67(s, 3H); 5,62(s, 1H) δ ppm.
- RMN(¹³C) :: 174,3(s); 167,2(s); 164,5(s); 135,6(s); 128,6(s); 106,8(d); 51,1(q); 39,8(t); 36,0(t); 35,0(s); 34,2(d); 32,8(t); 28,5(2q); 25,3(t); 19,7(q); 19,5(t); 16,9(2q) δ ppm.
- SM :: 322(0, M⁺), 234(7), 219(11), 137(42), 71(100).

Le composé (e) a été préparé selon le même procédé que celui décrit ci-dessus en utilisant comme réactif le chlorure de 2,2-diméthylpropionyle. Le composé (e) a été obtenu sous forme d'un mélange isomérique trans/cis (>9:1) : 2,6 g; rend. 82%.
- Eb. :: 140-145°/40 Pa.
- IR(CHCl₃) :: 2934, 1745, 1717, 1660, 1436, 1361, 1216, 1098.

Isomère trans :
- R_{f} (cyclohexane/AcOEt 9:1) :: 0,52.
- RMN(¹H) :: 1,02(s, 6H); 1,30(s, 9H); 1,42(2H); 1,57(2H); 1,66(s, 3H); 1,92(2H); 2,20(2H); 2,87(2H); 3,72(s, 3H); 5,62(s, 1H) δ ppm.
- RMN(¹³C) :: 175,8(s); 167,5(s); 166,2(s); 135,9(s); 128,6(s); 109,0(d); 51,3(q); 40,0(t); 39,3(s); 35,1(s); 32,9(t); 31,9(t); 28,5(2q); 27,1(3q); 25,7(t); 19,7(q); 19,5(t) δ ppm.
- SM :: 336(0, M⁺), 137(31), 95(12), 85(19), 57(100).

Isomère cis :
- R_{f} (cyclohexane/AcOEt 9:1) :: 0,44.
- RMN(¹H) :: 0,98(s, 6H); 3,67(s, 3H) δ ppm.

### Préparation du composé (f)

1,5 Ml (12,9 mM) de chlorure de benzoyle ont été ajoutés, à 2° sous atmosphère d'azote, goutte à goutte à une solution sous agitation de 2,5 g (9,4 mM) de 5-(2,6,6-triméthyl-1-cyclohexén-1-yl)-3-oxopentanoate de méthyle, 1,3 g (12,8 mM) de triéthylamine dans 30 ml de toluène contenant 0,04 g (0,3 mM) de 4-(N,N-diméthylamino)pyridine. Après 2,5 h à température ambiante, le mélange de réaction a été versé dans une solution aqueuse saturée de NH₄Cl et extrait à l'éther.
Les traitements usuels suivis de distillation ont fourni l'ester (f) désiré sous forme d'un mélange isomérique trans/cis (53:47) : 3,3 g; rend. 93%.
Une séparation chromatographique sur colonne a permi d'obtenir le composé (f) sous forme d'isomères trans et cis purs.

Isomère trans :
- Eb. (four à boules) :: 200-220°/8 Pa.
- R_{f} (cyclohexane/AcOEt 9:1) :: 0,43.
- IR(CHCl₃) :: 2932, 1735, 1661, 1437, 1361, 1261, 1203, 1159, 1104, 1067, 1028.
- RMN(¹H) :: 1,00(s, 6H); 1,40(2H); 1,55(2H); 1,62(s, 3H); 1,89(large t, J=7, 2H); 2,29(m, 2H); 3,01(m, 2H); 3,75(s, 3H); 5,83(s, 1H); 7,50(t, J=8, 2H); 7,63(large t, J=7, 1H); 8,10(large d, J=7,5, 2H) δ ppm.
- RMN(¹³C) :: 167,2(s); 166,2(s); 164,0(s); 135,9(s); 133,8(d); 130,1(2d); 129,2(s); 128,7(2d); 128,6(s); 109,5(d); 51,4(q); 39,9(t); 35,0(s); 32,9(t); 32,0(t); 28,5(2q); 25,7(t); 19,6(q); 19,5(t) δ ppm.
- SM :: 356(0, M⁺), 137(22), 105(100), 77(16).

Isomère cis :
- Eb. (four à boules) :: 200-220°/8 Pa.
- R_{f} (cyclohexane/AcOEt 9:1) :: 0,34.
- IR(CHCl₃) :: 2933, 1729, 1667, 1437, 1270, 1199, 1175, 1139, 1083, 1066, 1026.
- RMN(¹H) :: 0,99(s, 6H); 1,41(2H); 1,56(2H); 1,64(s, 3H); 1,90(large t, J=7, 2H); 2,30(m, 2H); 2,47(m, 2H); 3,61(s, 3H); 5,74(s, 1H); 7,48(t, J=8, 2H); 7,61(t, J=7, 1H); 8,14(large d, J=7,5, 2H) δ ppm.
- RMN(¹³C) :: 164,4(s); 163,9(s); 163,8(s); 135,5(s); 133,5(d); 130,2(2d); 129,5(s); 128,7(s); 128,6(2d); 107,0(d); 51,3(q); 39,8(t); 36,1(t); 35,0(s); 32,8(t); 28,5(2q); 25,2(t); 19,8(q); 19,4(t) δ ppm.
- SM :: 356(0, M⁺), 241(3), 137(9), 105(100), 77(19).

Le 5-(2,6,6-triméthyl-1-cyclohexén-1-yl)-3-oxopentanoate de méthyle, utilisé comme produit de départ dans les procédés ci-dessus peut être obtenu suivant la méthode décrite par G. Büchi et H. Wüest, Helv. Chim. Acta 72, 996(1989).

### Exemple 2

On a chargé 1060 g d'acide sulfurique aqueux à 98% et 2 l de toluène dans un réacteur tricol équipé d'un agitateur mécanique, d'un thermomètre, d'un réfrigérant et d'une ampoule d'introduction. Le mélange a été refroidi à 3-4°, puis on y a introduit goutte à goutte 1060 g (3,14 moles) de 7,11-diméthyl-3-(2,2-diméthyl-propionoxy)-dodéca-2,6,10-triénoate de méthyle. Pendant l'addition on a veillé à ne pas dépasser 15°, puis le mélange a été maintenu sous agitation pendant 1,5 h à 5-10°. 1 Kg de glace pilée a été ensuite versée graduellement dans le mélange réactionnel tandis que la température était maintenue à une valeur inférieure à 20°. Après décantation, le mélange a été lavé avec une solution aqueuse saturée de bicarbonate de sodium et extrait au toluène.
L'étape suivante de décarboxylation a été effectuée par traitement avec de la soude. Pour une quantité de 1060 g (3,13 moles) de perhydro-5,5,8a-triméthyl-3-carbométhoxy-2-naphtalénone on a utilisé 2720 g d'une solution aqueuse de NaOH à 30%.
On a ainsi obtenu 423,5 g (1,66 moles) de perhydro-5,5,8a-triméthyl-2-naphtalénone dont le contenu en l'isomère trans était de 76%.
D'autres réactions de cyclisation acide du 7,11-diméthyl-3-(2,2-diméthyl-propionoxy)-dodéca-2,6,10-triénoate de méthyle ont été effectuées en remplaçant l'acide sulfurique par l'acide formique, l'acide phosphorique et le BF₃ sous forme de trifluoroboroéthérate.
L'ester méthylique de départ a été préparé comme décrit ci-après à partir d'une fraction de géranylacétone riche en l'isomère trans.
a. 24 G (0,55 M) d'hydrure de sodium (dispersion dans l'huile à 55%) ont été ajoutés à 20° sous azote à une solution de 135 g (1,5 M) de diméthylcarbonate dans 500 ml d'un mélange de toluène/N-méthylpyrrolidone (9:1). Le mélange obtenu à été chauffé à reflux (temp. bain : 100°) puis, pendant 1 h, on y a ajouté une solution de 0,5 M de géranylacétone dans 135 g (1,5 M) de diméthylcarbonate après quoi le mélange de réaction résultant a été laissé à reflux pendant 10 minutes. Le tout a été versé dans une solution aqueuse à 10% de NH₄Cl saturée avec du NaCl.
   Une extraction à l'éther, suivie des traitements usuels des extraits organiques combinés et de distillation fractionnée à fourni le 3-oxo-7,11-diméthyl-dodéca-6,10-diénoate de méthyle.
b. Un mélange de 796 g du céto-ester obtenu dans 200 ml d'éther de pétrole 80-100 comme indiqué ci-dessus (3,16 M) et 349,5 g (3,46 M) de triéthylamine a été chargé dans un réacteur tricol équipé d'un thermomètre et d'une ampoule d'introduction ainsi que d'un agitateur mécanique. Au mélange résultant chauffé à 70°, on a introduit, en 2 h tout en agitant, 388 g (3,22 M) de chlorure de pivaloyle.
   Après refroidissement, le mélange a été lavé avec deux fractions de 900 ml chacune d'eau et la partie organique à été concentrée pour fournir 1060 g de 7,11-diméthyl-3-(2,2-diméthyl-propionoxy)-dodéca-2,6,10-triénoate de méthyle dont les caractères analytiques étaient les suivants :
   - RMN(¹H, 360MHz, CDCl₃) :: 1,27 et 1,28(2s, 9H); 1,60 et 1,68(2 large s, 9H); 1,94-2,08(m, 4H); 2,21(q, J=7, 2H); 2,82 et 2,84(2 tr, J=7, 2H); 3,71(s, 3H); 5,08 et 5,15(2 large t, J=7, 2H); 5,65(s, 1H) δ ppm.
   - RMN(¹³C, 90,5MHz, CDCl₃) :: 15,9(q); 17,7(2q); 23,4(q); 25,3(t); 25,7(2q); 26,6(t); 26,7(t); 27,0(6q); 31,2(t); 31,5(t); 31,9(t); 39,3(2s); 39,7(2t); 51,3(2q); 109,5(2d); 122,5(2d); 123,4(d); 124,3(d); 131,4(s); 131,6(s); 136,6(2s); 166,2(s); 166,3(s); 167,4(2s); 175,8(2s) δ ppm.
   - SM :: 336(0, M⁺), 57(100), 69(36), 85(17), 41(17), 81(10), 109(7), 123(6), 67(6), 151(5), 101(5), 136(4), 95(4).

## Revendications

1. Procédé pour la préparation de perhydro-5,5,8a-triméthyl-2-naphtalénone essentiellement sous la forme isomérique de formule 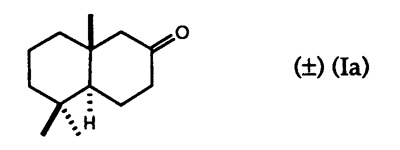 caractérisé en ce qu'on traite avec un agent de cyclisation acide
a. un ester carboxylique de formule 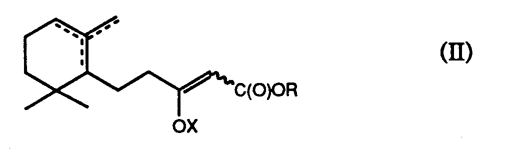 possédant une double liaison dans l'une des position indiquées par les pointillés et dans laquelle le symbole R définit un groupe alkyle C₁-C₆, X sert à désigner un radical monovalent de formule P(O)(OR¹)₂ ou C(O)R², R¹ étant un groupe alkyle C₁-C₆ et R² représentant soit un groupe alkyle linéaire ou ramifié C₁-C₆, soit un groupe phényle substitué ou non, et dans laquelle la ligne ondulée sert à définir une liaison C-C de configuration cis ou trans,
ou
b. un ester carboxylique de formule 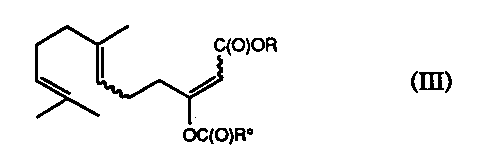 dans laquelle les lignes ondulées et le symbole R sont définis comme indiqué plus haut, et R⁰ représente un radical alkyle C₃-C₆, de préférence ramifié,
et qu'on décarboxyle ensuite le produit de cyclisation ainsi obtenu au moyen d'un traitement avec une base.

2. Procédé selon la revendication 1, caractérisé en ce que l'agent de cyclisation est un acide protique, minéral ou organique, ou un acide de type dit de Lewis.

3. Procédé selon la revendication 2, caractérisé en ce qu'on utilise comme acide protique l'acide sulfurique, phosphorique, chlorosulfonique, p-toluènesulfonique ou formique et comme acide de Lewis le BF₃.

4. Procédé selon la revendication 1, caractérisé en ce qu'on utilise comme produit de départ un ester carboxylique de préférence sous sa forme isomérique trans, ou sous forme d'un mélange contenant ledit isomère accompagné de quantités inférieures de son isomère cis.

5. Procédé selon la revendication 1, caractérisé en ce qu'on utilise comme ester carboxylique le
5-(2,6,6-triméthyl-1-cyclohexén-1-yl)-3-(diéthoxyphosphoroxy)-pent-2-énoate de méthyle,
5-(2,6,6-triméthyl-1-cyclohexén-1-yl)-3-propionoxypent-2-énoate de méthyle,
5-(2,6,6-triméthyl-1-cyclohexén-1-yl)-3-(2-méthylpropionoxy)-pent-2-énoate de méthyle,
5-(2,6,6-triméthyl-1-cyclohexén-1-yl)-3-(2,2-diméthylpropionoxy)-pent-2-énoate de méthyle,
5-(2,6,6-triméthyl-1-cyclohexén-1-yl)-3-benzoyloxypent-2-énoate de méthyle, ou le
7,11-diméthyl-3-(2,2-diméthyl-propionoxy)-dodéca-2,6,10-triénoate de méthyle.

6. Procédé selon la revendication 5, caractérisé en ce que lesdits esters carboxyliques sont essentiellement sous leur forme isomérique trans.

7. Procédé selon la revendication 1, caractérisé en ce que l'agent de cyclisation acide est employé à raison d'au moins deux équivalents par rapport à l'ester carboxylique de départ.

8. Procédé selon la revendication 1, caractérisé en ce que la réaction de cyclisation s'effectue à une température comprise entre 0° et 25° C.

9. Procédé selon la revendication 1, caractérisé en ce que la décarboxylation est effectuée au moyen d'un traitement avec un hydroxyde d'un métal alcalin, de préférence le KOH en solution aqueuse-alcoolique ou le NaOH.

10. Un ester carboxylique de formule
a. 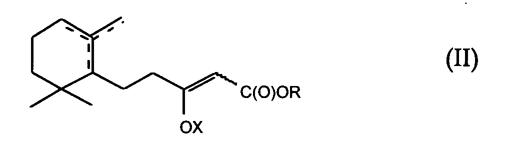 possédant une double liaison dans l'une des position indiquées par les pointillés et dans laquelle le symbole R définit un groupe alkyle C₁-C₆, X sert à désigner un radical monovalent de formule P(O)(OR¹)₂ ou C(O)R², R¹ étant un groupe alkyle C₁-C₆ et R² représentant soit un groupe alkyle linéaire ou ramifié C₁-C₆, soit un groupe phényle substitué ou non, et dans laquelle la ligne ondulée sert à définir une liaison C-C de configuration cis ou trans,
ou
b. 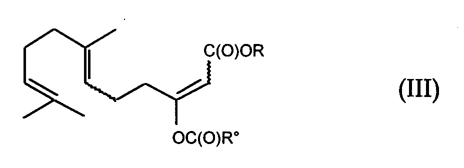 dans laquelle les lignes ondulées et le symbole R sont définis comme indiqués plus haut, et R⁰ représente un radical alkyle C₃-C₆, de préférence ramifié, à l'exclusion du (Z)-5-(2,6,6-triméthyl-1-cyclohexén-1-yl)-3-(diéthoxyphosphoroxy)-pent-2-énoate de méthyle.

11. A titre d'ester carboxylique selon la revendication 10, le
(E)-5-(2,6,6-triméthyl-1-cyclohexén-1-yl)-3-(diéthoxyphosphoroxy)-pent-2-énoate de méthyle,
5-(2,6,6-triméthyl-1-cyclohexén-1-yl)-3-propionoxypent-2-énoate de méthyle,
5-(2,6,6-triméthyl-1-cyclohexén-1-yl)-3-(2-méthylpropionoxy)-pent-2-énoate de méthyle,
5-(2,6,6-triméthyl-1-cyclohexén-1-yl)-3-(2,2-diméthylpropionoxy)-pent-2-énoate de méthyle,
5-(2,6,6-triméthyl-1-cyclohexén-1-yl)-3-benzoyloxypent-2-énoate de méthyle, ou le
7,11-diméthyl-3-(2,2-diméthyl-propionoxy)-dodéca-2,6,10-triénoate de méthyle.

## Claims

1. A process for the preparation of perhydro-5,5,8a-trimethyl-2-naphthalenone essentially in its isomeric form of formula 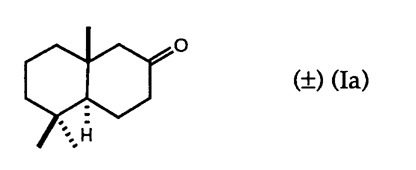 characterized in that there is treated with an acidic cyclization agent
a. a carboxylic ester of formula 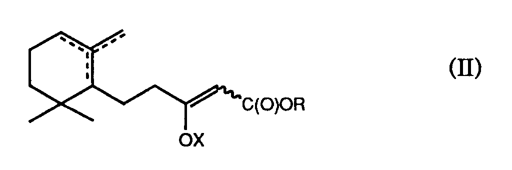 having a double bond in one of the positions indicated by the dotted lines and wherein symbol R designates a C₁ - C₆ alkyl group, X stands for a monovalent radical of formula P(O)(OR¹)₂ or C(O)R², R¹ representing a C₁ - C₆ alkyl group and R² either a linear or branched C₁ - C₆ alkyl group or a substituted or unsubstituted phenyl group, and wherein the wavy line represents a C - C bond of cis or trans configuration,
or
b. a carboxylic ester of formula 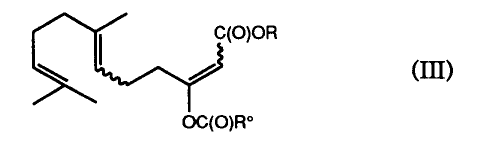 wherein the wavy lines and symbol R are defined as above, and R⁰ represents a C₃ - C₆ alkyl radical, preferably branched, and in that subsequently the thus obtained cyclization product is decarboxylated by treating it with a base.

2. A process according to claim 1, characterized in that the cyclization agent is a protic mineral or organic acid or a Lewis type acid.

3. A process according to claim 2, characterized in that the protic acid is sulfuric, phosphoric, chlorosulfonic, p-toluenesulfonic or formic acid and the Lewis type acid is BF₃.

4. A process according to claim 1, characterized in that there is used as starting product a carboxylic ester preferably in its trans isomeric form, or in the form of a mixture containing said trans isomer together with smaller amounts of its cis isomer.

5. A process according to claim 1, characterized in that there is used as the carboxylic ester:
methyl 5-(2,6,6-trimethyl-1-cyclohexen-1-yl)-3-(diethoxyphosphoroxy)pent-2-enoate,
methyl 5-(2,6,6-trimethyl-1-cyclohexen-1-yl)-3-propionoxypent-2-enoate,
methyl 5-(2,6,6-trimethyl-1-cyclohexen-1-yl)-3-(2-methylpropionoxy)pent-2-enoate,
methyl 5-(2,6,6-trimethyl-1-cyclohexen-1-yl)-3-(2,2-dimethylpropionoxy)pent-2-enoate,
methyl 5-(2,6,6-trimethyl-1-cyclohexen-1-yl)-3-benzoyl-oxypent-2-enoate, or
methyl 7,11-dimethyl-3-(2,2-dimethylpropionoxy)dodeca-2,6,10-trienoate.

6. A process according to claim 5, characterized in that the carboxylic esters are essentially in their trans isomeric configuration.

7. A process according to claim 1, characterized in that the acid cyclization agent is used in a proportion of at least 2 equivalents relative to the carboxylic ester starting material.

8. A process according to claim 1, characterized in that the cyclization is effected at a temperature comprised between 0° and 25°C.

9. A process according to claim 1, characterized in that the decarboxylation is effected by means of a treatment with an alkali metal hydroxide, preferably KOH in an aqueous-alcoholic solution or NaOH.

10. A carboxylic ester of formula
a. 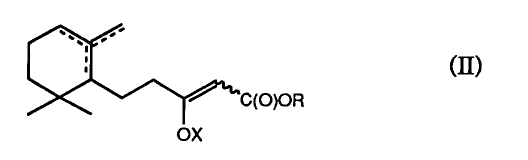 having a double bond in one of the positions indicated by the dotted lines and wherein symbol R designates a C₁ - C₆ alkyl group, X stands for a monovalent radical of formula P(O)(OR¹)₂ or C(O)R², R¹ representing a C₁ - C₆ alkyl group and R² either a linear or branched C₁ - C₆ alkyl group or a substituted or unsubstituted phenyl radical, and wherein the wavy line represents a C - C bond of cis or trans configuration,
or
b. 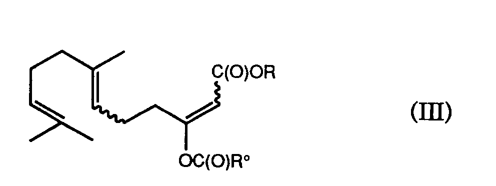 wherein the wavy lines and symbol R are defined as above, and R⁰ represents a C₃ - C₆ alkyl radical, preferably branched, provided that methyl (Z)-5-(2,6,6-trimethyl-1-cyclohexen-1-yl)-3-(diethoxyphosphoroxy)-pent-2-enoate is excluded.

11. As a carboxylic ester according to claim 10,
methyl 5-(2,6,6-trimethyl-1-cyclohexen-1-yl)-3-(diethoxyphosphoroxy)pent-2-enoate,
methyl 5-(2,6,6-trimethyl-1-cyclohexen-1-yl)-3-propionoxypent-2-enoate,
methyl 5-(2,6,6-trimethyl-1-cyclohexen-1-yl)-3-(2-methylpropionoxy)pent-2-enoate,
methyl 5-(2,6,6-trimethyl-1-cyclohexen-1-yl)-3-(2,2-dimethyl-propionoxy)pent-2-enoate,
methyl 5-(2,6,6-trimethyl-1-cyclohexen-1-yl)-3-benzoyl-oxypent-2-enoate, or
methyl 7,11-dimethyl-3-(2,2-dimethylpropionoxy)dodeca-2,6,10-trienoate.

## Patentansprüche

1. Verfahren zur Herstellung von Perhydro-5,5,8a-trimethyl-2-naphthalinon, welches sich im wesentlichen in der isomeren Form der Formel 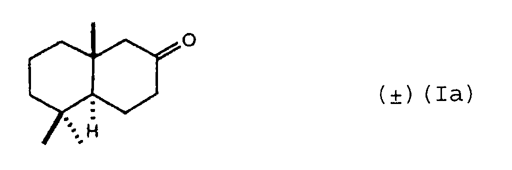 befindet, dadurch gekennzeichnet, dass man
a. einen Carbonsäureester der Formel 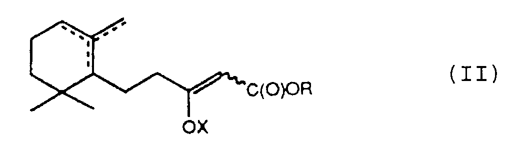 welcher eine Doppelbindung in einer der durch die punktierten Linien angegebenen Stellungen besitzt und worin das Symbol R eine C₁-C₆-Alkylgruppe bedeutet, X einen einwertigen Rest der Formel P(O)(OR¹)₂ oder C(O)R² bezeichnet, wobei R¹ für eine C₁-C₆-Alkylgruppe steht und R² entweder eine geradekettige oder verzweigte C₁-C₆-Alkylgruppe bedeutet oder für eine substituierte oder unsubstituierte Phenylgruppe steht, und worin die Wellenlinie eine C-C-Bindung mit Cis- oder Trans-Konfiguration bezeichnet,
oder
b. einen Carbonsäureester der Formel 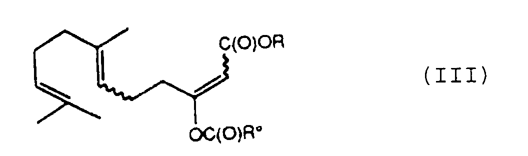 worin die Wellenlinien und das Symbol R die obige Bedeutung besitzen und R⁰ eine vorzugsweise verzweigte C₃-C₆-Alkylgruppe bedeutet
mit einem sauren Cyclisierungsmittel behandelt und das so erhaltene Cyclisierungsprodukt anschliessend durch Behandlung mit einer Base decarboxyliert.

2. Verfahren gemäss Patentanspruch 1, dadurch gekennzeichnet, dass das Cyclisierungsmittel eine anorganische oder eine organische Protonensäure oder eine Säure vom Lewis-Typus ist.

3. Verfahren gemäss Patentanspruch 2, dadurch gekennzeichnet, dass man als Protonensäure die Schwefelsäure, Phosphorsäure, Chlorsulfonsäure, p-Toluolsulfonsäure oder Ameisensäure und als Lewis-Säure BF₃ verwendet.

4. Verfahren gemäss Patentanspruch 1, dadurch gekennzeichnet, dass man als Ausgangsverbindung einen Carbonsäureester, vorzugsweise in Form seines Trans-Isomeren, verwendet oder in Form einer Mischung, welche dieses genannte Isomere, begleitet von geringeren Mengen seines Cis-Isomeren, enthält.

5. Verfahren gemäss Patentanspruch 1, dadurch gekennzeichnet, dass man als Carbonsäureester verwendet das
Methyl 5-(2,6,6-trimethyl-1-cyclohexen-1-yl)-3-(diethoxyphosphoroxy)-pent-2-enoat,
Methyl 5-(2,6,6-trimethyl-1-cyclohexen-1-yl)-3-propionoxypent-2-enoat,
Methyl 5-(2,6,6-trimethyl-1-cyclohexen-1-yl)-3-(2-methylpropionoxy)-pent-2-enoat
Methyl 5-(2,6,6-trimethyl-1-cyclohexen-1-yl)-3-(2,2-dimethylpropionoxy)-pent-2-enoat,
Methyl 5-(2,6,6-trimethyl-1-cyclohexen-1-yl)-3-benzoyloxypent-2-enoat oder das
Methyl 7,11-dimethyl-3-(2,2-dimethyl-propionoxy)-dodeca-2,6,10-trienoat.

6. Verfahren gemäss Patentanspruch 5, dadurch gekennzeichnet, dass die genannten Carbonsäureester im wesentlichen in Form ihrer Trans-Isomeren vorliegen.

7. Verfahren gemäss Patentanspruch 1, dadurch gekennzeichnet, dass das saure Cyclisierungsmittel in einer Menge von mindestens zwei Äquivalenten bezogen auf den Ausgangscarbonsäureester verwendet wird.

8. Verfahren gemäss Patentanspruch 1, dadurch gekennzeichnet, dass die Cyclisierungsreaktion bei einer Temperatur von 0° bis 25°C erfolgt.

9. Verfahren gemäss Patentanspruch 1, dadurch gekennzeichnet, dass die Decarboxylierung durch Behandlung mit einem Alkalimetallhydroxyd vorzugsweise KOH in wässrig-alkoholischer Lösung oder NaOH erfolgt.

10. Ein Carbonsäureester der Formel
a. 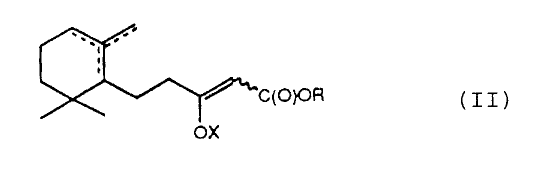 welcher eine Doppelbindung in einer der durch die punktierten Linien angegebenen Stellungen besitzt und worin das Symbol R eine C₁-C₆-Alkylgruppe bedeutet, X einen einwertigen Rest der Formel P(O)(OR¹)₂ oder C(O)R² bezeichnet, R¹ für eine C₁-C₆-Alkylgruppe steht und R² entweder eine geradekettige oder verzweigte C₁-C₆-Alkylgruppe bedeutet oder für eine substituierte oder unsubstituierte Phenylgruppe steht, und worin die Wellenlinie eine C-C-Bindung mit Cis- oder Trans-Konfiguration bezeichnet,
oder
b. 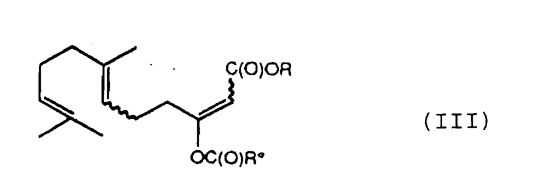 worin die Wellenlinien und das Symbol R die obige Bedeutung besitzen und R⁰ eine vorzugsweise verzweigte C₃-C₆-Alkylgruppe bedeutet, mit Ausnahme von (Z)-Methyl 5-(2,6,6-trimethyl-1-cyclohexen-1-yl)-3-(diethoxyphosphoroxy)-pent-2-enoat.

11. Carbonsäureester gemäss Patentanspruch 10, nämlich
(E)-Methyl 5-(2,6,6-trimethyl-1-cyclohexen-1-yl)-3-(diethoxyphosphoroxy)-pent-2-enoat,
Methyl 5-(2,6,6-trimethyl-1-cyclohexen-1-yl)-3-propionoxy-pent-2-enoat,
Methyl 5-(2,6,6-trimethyl-1-cyclohexen-1-yl)-3-(2-methylpropionoxy)-pent-2-enoat
Methyl 5-(2,6,6-trimethyl-1-cyclohexenl-yl)-3-(2,2-dimethylpropionoxy)-pent-2-enoat,
Methyl 5-(2,6,6-trimethyl-1-cyclohexen-1-yl)-3-benzoyloxypent-2-enoat oder das
Methyl 7,11-dimethyl-3-(2,2-dimethyl-propionoxy)-dodeca-2,6,10-trienoat.
